# EUROPEAN PATENT APPLICATION

(11) **EP 3 957 322 A1**
(43) Date of publication of application: **23.02.2022**
(21) Application number: 20191751.5
(22) Date of filing: 19.08.2020
(51) Int. Cl.: A61K 39/12

(54) **RECOMBINANT POXVIRIDAE VECTOR EXPRESSING CO-STIMULATORY MOLECULES**

(71) Applicant: Eberhard Karls Universität Tübingen Medizinische Fakultät, 72074 Tübingen (DE)
(72) Inventor: Müller, Melanie, 72076 Tübingen (DE); Amann, Ralf, 72070 Tübingen (DE)
(74) Representative: Witte, Weller & Partner Patentanwälte mbB

(57) **Abstract**

The present invention relates to a recombinant *Poxviridae* vector expressing a co-stimulatory molecule, a cell containing the recombinant *Poxviridae* vector, a pharmaceutical composition containing the recombinant *Poxviridae* vector according to the invention and/or the cell according to the invention, and a method for the treatment of a living being in need of a vaccination with an antigen.

## Description

The present invention relates to a recombinant *Poxviridae* vector expressing a co-stimulatory molecule, a cell containing the recombinant *Poxviridae* vector, a pharmaceutical composition containing the recombinant *Poxviridae* vector according to the invention and/or the cell according to the invention, and a method for the treatment of a living being in need of a vaccination with an antigen.

Viral vectors are used in the biotechnology to introduce genetic material into target cells. The introduced genetic material often encodes foreign genes which serve for the production of a recombinant protein. For this reason viral vectors are a significant platform technology, in particular for the production of recombinant vaccines which are increasingly used, along with the traditional prevention of infectious diseases, also for the development of new, innovative therapeutic concepts, such as e.g. the therapeutic tumor immunization.

*Poxviridae* or poxviruses is a family of viruses where humans, vertebrates, and arthropods serve as natural hosts. There are currently 83 species in this family, divided among 22 genera, which are divided into two subfamilies. Four genera of poxviruses may infect humans: orthopoxvirus, parapoxvirus, yatapoxvirus, mollus-cipoxvirus. Orthopox: smallpox virus (variola), vaccinia virus, cowpox virus, monkeypox virus; Parapox: Orf virus, pseudocowpox, bovine papular stomatitis virus; Yatapox: tanapox virus, yaba monkey tumor virus; Molluscipox: molluscum contagiosum virus (MCV). The most common are vaccinia (seen on Indian subcontinent) and molluscum contagiosum, but monkeypox infections are rising (seen in west and central African rainforest countries). The prototypical poxvirus is vaccinia virus, known for its role in the eradication of smallpox. *Poxviridae* viral particles (virions) are generally enveloped (external enveloped virion), though the intracellular mature virion form of the virus, which contains different envelope, is also infectious. The vaccinia virus is an effective tool for foreign protein expression, as it elicits a strong host immune-response. The vaccinia virus enters cells primarily by cell fusion, although currently the receptor responsible is unknown.

The Orf virus (ORFV) belongs to the family of poxviruses and has a variety of characteristics which makes it interesting for the production of recombinant vaccines and prefers it over other technologies. It is the prototype of the genus of the parapoxviruses and belongs to the family of the *Poxviridae.* ORFV are enveloped, complex dsDNA viruses having a morphology that reminds of a ball of wool and have an average size of approx. 260 x 160 nm. They have a linear DNA genome with high GC content and a size of approx. 130 to 150 kbp, the central region of which is delimited on both sides by ITR regions ("inverted terminal repeats") and ends in a hair-pin structure, which covalently links both DNA single strands to each other. In the central region of the genome there are predominantly genes which are mainly essential for the viral replication and morphogenesis and which are highly conserved among the pox viruses. In contrast, in the ITR regions there are so-called non-conserved virulence genes which significantly determine the host range, the pathogenicity and the immunomodulation and, therefore, characterize the virus.

In comparison to orthopoxviruses ORFV is characterized by a very narrow natural host tropism which includes sheep and goat. As a consequence, an inhibiting "preimmunity" against the vector in humans, which is caused by a natural infection, as it can be observed in the most common viral vectors of the vaccinia and adenoviruses, can be almost excluded. Furthermore, the exceptionally weak and short-lived ORFV specific vector immunity allows a very effective booster and/or refreshing vaccination or immunization with ORFV based vaccines which are directed against further pathogens.

While ORFV derived vectors are generally promising tools for the production of viral vector-based vaccines the immune responses induced when administering them into a living being are quite often not strong enough. Currently known ORFV derived vectors, therefore, do often not induce such a robust immune response which ensures the elimination of the disease-causing agent.

It is, therefore, a problem underlying the invention to provide an improved recombinant *Poxviridae* vector by means of which the problems of the current vectors are avoided or at least reduced. In particular, a recombinant *Poxviridae* vector should be provided which, after being administered to a living being, induces a strong immune stimulating reaction which renders the use of adjuvants redundant.

This problem is solved by the provision of a recombinant *Poxviridae* vector, which comprises the following:
- at least one first nucleotide sequence encoding and expressing a co-stimulatory molecule, and
- at least one promoter controlling the expression of the first nucleotide sequence.

According to the invention *Poxviridae* refers to all viruses and virus strains belonging to the family of poxviruses, in particular falling under the species of *Parapoxvirus ovis* such as the ORF virus (ORFV), in particular the strain D1701.

According to the invention a 'recombinant *Poxviridae* vector' refers to a vector based on the poxvirus genome which is configured for the transport and/or the expression of a foreign gene, such as an antigen, in(to) biological cells. According to the invention the 'recombinant *Poxviridae* vector' includes a 'recombinant *Poxviridae*-derived vector'.

According to the invention a 'co-stimulatory molecule' refers to a mammalian and in particular human, cow, pig, sheep, fowl, dog, cat horse protein that has to be activated as a second receptor in order to trigger an adaptive immune response. Often it is also the other way around, i.e. the co-stimulatory molecule causes a receptor or ligand to be activated, which then leads to an improved immune response. Examples of co-stimulatory molecules in B cells are CD40 and its ligand CD40L, or CR2 and one of its ligands iC3b, C3dg or C3d. Co-stimulatory molecules in T cells are CD28 and its ligands CD80 (B7-1) and CD86 (B7-2), as well as ICOS and its ligand ICOSL (B7-H2).

According to the invention the 'nucleotide sequence' refers to a nucleic acid comprising concatenated nucleotides and may be a DNA or RNA.

The first nucleotide sequence encoding and expressing the co-stimulatory molecule can be integrated at any position in the *Poxviridae* vector which does not or not significantly disturb the vector's replication capacity.

According to the invention a 'promoter' refers to such a nucleic acid section which allows the regulated expression of the co-stimulatory molecule in the *Poxviridae* vector of the invention. Preferably it refers to a *Poxviridae* promoter, i.e. a promoter existing in the wild type *Poxviridae* genome or a promoter derived therefrom, or an artificial promoter, such as a poxvirus promoter, ORF promoter, CMV promoter etc. Typically, but not necessarily, the promotor is located in close proximity and upstream to the first nucleotide sequence.

The inventors have realized that the *Poxviridae* vector according to the invention is, due to the expression of a co-stimulatory molecule, capable to induce a strong immune response when administered to a living being. It can therefore be used, e.g., as a vaccine vector which is clearly superior to the vaccine vectors used so far in the state of the art.

In an embodiment of the recombinant *Poxviridae* vector according to the invention the co-stimulatory molecule is CD40L.

This measure has the advantage that such a co-stimulating molecule is used, which leads to a particularly good improved immunogenicity of the vector. CD40L does not occur naturally in APCs but activates them by binding to CD40. A special feature of the inventors' approach is that, since the ORFV vector is taken up by the APCs, the APCs that have taken up the vector are activated (quasi an autocrine activation). This has the advantage that one now has an increasingly activated APC that also presents antigens from cancer or infectious agents and thus induces a stronger immune response.

According to the invention 'CD40L' or 'cluster of differentiation' (CD) 40L, also called CD40 ligand or CD154, is a protein that is naturally primarily expressed on activated T cells and is a member of the TNF superfamily of molecules. It binds to CD40 on antigen-presenting cells (APC), which leads to many effects depending on the target cell type. In total CD40L has three binding partners: CD40, α5β1 integrin and αIIbβ3. CD40L acts as a costimulatory molecule and is particularly important on a subset of T cells called T follicular helper cells (TFH cells). On TFH cells, CD40L promotes B cell maturation and function by engaging CD40 on the B cell surface and therefore facilitating cell-cell communication. A defect in this gene results in an inability to undergo immunoglobulin class switching and is associated with hyper IgM syndrome. Absence of CD40L also stops the formation of germinal centers and therefore prohibiting antibody affinity maturation, an important process in the adaptive immune system. The human variant of CD40L has the Gene ID 959 (HGNC:11935), the mouse variant has the Gene ID 21947 (MGI:88337).

In another embodiment of the recombinant *Poxviridae* vector according to the invention the co-stimulatory molecule is CD80.

This measure has the advantage that another co-stimulating molecule is used, which leads to a particularly well immunogenicity of the vector.

According to the invention 'CD80', also called B7-1, is a B7, type I membrane protein that is in the immunoglobulin superfamily, with an extracellular immunoglobulin constant-like domain and a variable-like domain required for receptor binding. It is closely related to CD86, another B7 protein (B7-2), and often works in tandem, binding to the same receptors to prime T cells. Naturally, CD80 can be found on the surface of various immune cells including B cells, monocytes and antigen-presenting cells (APCs) such as dendritic cells and is the receptor for the proteins CD28 (for autoregulation and intercellular association) and CTLA-4 (for attenuation of regulation and cellular disassociation) found on the surface of T-cells. CD80 binds to CD28 and CTLA-4 with lower affinity and fast binding kinetics, allowing for quick interactions between the communicating cells. This interaction results in an important costimulatory signal in the immunological synapse between antigen-presenting cells, B-cells, dendritic cells and T-cells that result in T and B-cell activation, proliferation and differentiation. CD80 is an especially important component in dendritic cell licensing and cytotoxic T-cell activation. When the major histocompatibility complex class II (MHC class II)- peptide complex on a dendritic cell interacts with the receptor on a T helper cell, CD80 is up-regulated, licensing the dendritic cell and allowing for interaction between the dendritic cell and CD 8⁺ T-cells via CD28. This helps to signal the T-cell differentiation into a cytotoxic T-cell. The human variant of CD80 has the Gene ID 941 (HGNC:1700), the mouse variant has the Gene ID 12519 (MGI:101775).

In an embodiment of the invention the recombinant *Poxviridae* vector additionally comprises the following:
- at least a second nucleotide sequence encoding and expressing a co-stimulatory molecule, and
- at least one promoter controlling the expression of the second nucleotide sequence.

This measure has the advantage that due to the second co-stimulatory molecule the immunogenicity of the recombinant *Poxviridae* vector can be even further improved.

The features, characteristics and particularities of the first nucleotide sequence and the promoter associated therewith apply to the second nucleotide sequence and its associated promoter mutatis mutandis.

In another embodiment of the recombinant *Poxviridae* vector of the invention the co-stimulatory molecule is CD40L and/or CD80.

According to this embodiment the recombinant *Poxviridae* vector encodes CD40L or CD80 but, preferably, it encodes both CD40L via the first or the second nucleotide sequence and CD80 via the second of the first nucleotide sequence. The expression of both CD40L and CD80 ensures an even improved immunogenicity of the vector according to the invention.

According to the invention the recombinant *Poxviridae* vector is a recombinant Parapoxvirus vector (Parapoxvirus-derived vector), preferably a recombinant Orf virus (ORFV) vector (ORFV-derived vector), further preferably the ORFV is of the strain D1701.

This measure has the advantage that such a vector is used which is particularly suited for the purposes of the invention. In particular the ORFV strain D1701 is attenuated and only causes asymptotic infections in the host. According to the invention all variants of D1701 are covered, including D1701-B and D1701-V. The ORFV vector of the strain D1701 is well characterized in the art, e.g. in Rziha et al. (2019), Genomic Characterization of Orf Virus Strain D1701-V (Parapoxvirus) and Development of Novel Sites for Multiple Transgene Expression, Viruses 11, 125, pp. 1-26. The content thereof is herein incorporated by reference.

In a further embodiment of the recombinant *Poxviridae* vector of the invention the first and/or the second nucleotide sequence is located in the VEGF locus.

According to the findings of the inventors the insertion of the first and/or the second nucleotide sequence in the VEGF locus ensures a strong expression of the co-stimulatory molecule while, at the same time, an efficient replication of the *Poxviridae* vector in the host cell. In the ORFV strain D1701 the VEGF locus is located at genome's sections comprising: (i) the restriction fragments Hind*III* fragment H', Bam*HI* fragment A, Kpn*I* fragment F', Eco*RI* fragment C' and (ii) the gene/ORF 132.

The restriction map of the ORFV is exemplarily shown for the strain D1701 in Cottone et al. (1998), Analysis of genomic rearrangement and subsequent gene deletion of the attenuated Orf virus strain D1701, Virus Research, Vol. 56, pp. 53-67. The content of this publication is a component of the present disclosure. Here the indications Hind*III* fragment H', Bam*HI* fragment A, Kpn*I* fragment F', Eco*RI* fragment C' mean that the insertion locus extends from the Hind*III* fragment H' up to the Eco*RI* fragment C' fragment.

In another embodiment of the invention the recombinant *Poxviridae* vector additionally comprises the following:
- at least one third nucleotide sequence encoding and expressing an antigen, and
- at least one promoter controlling the expression of the third nucleotide sequence.

This measure provides the constructive preconditions for the use of the recombinant *Poxviridae* vector as a vaccine vector.

According to the invention, an 'antigen' refers to a gene or open reading frame (ORF) or protein encoded thereby, which does not originate from the *Poxviridae* genome, e.g. a foreign gene or protein, and which, optionally after being processed, can be bound to by an antigen-specific antibody (Ab), a T cell receptor (TCR) or a B cell antigen receptor (BCR). The presence of such antigen in the mammalian's body typically triggers an immune response.

In this context 'an antigen' means that one or more antigens are encoded by the third nucleotide sequence, e.g. 1, 2, 3, 4, 5, 6, 7, 8, 9, 10, etc. different antigens.

In an embodiment of the recombinant *Poxviridae* vector according to the invention the third nucleotide sequence is located in the Del2 locus.

The inventors have found out that, while in principle all insertion sites of the *Poxviridae* vector are appropriate, the Del2 is particularly well suited for a stable expression of the antigen.

In the ORFV strain D1701 the Del2 locus is located at genome's sections comprising: (i) the restriction fragments Hind*III*-Fragment G/D, Kpn*I*-Fragment B, Bam*HI*-Fragment A, Eco*RI*-Fragment D (i.e. it extends from the Hind*III* fragment G/D up to the Eco*RI*-fragment D); (ii) the genes/ORFs 114, 115, 116, 117 (GIF); and (iii) the nucleotide (nt) positions nt 15.660 ± 100 to nt 17.850 ± 100. In the document Rziha *et al.* (2019, *l.c.*) the Del2 locus is designated as 'deletion site D'. In document WO 2017/013023 Del2 is referred to as 'IL3'.

In an embodiment of the invention of the recombinant ORFV vector the antigen is selected from the following group:
- viral antigen, preferably
   rabies virus antigen, including glycoprotein (RabG);
   influenza A antigen, including nucleoprotein (NP), hemagglutinin (HA), neuraminidase (NA);
   SARS-CoV-2 antigen, including spike protein (S) or fragments thereof;
- tumor antigen, preferably viral tumor antigen, including HPV selective viral tumor antigen;
- tumor associated antigen, including viral tumor associated antigen, including HPV selective viral tumor-associated antigen;
- parasitic antigen, preferably plasmodium antigen;
- cytokine.

This measure has the advantage that especially important antigens, in particular for the generation of vaccines, are expressible via the recombinant *Poxviridae* virus according to the invention.

Another subject-matter of the present invention relates to a biological cell, preferably a mammalian cell, further preferably a Vero cell, containing the recombinant *Poxviridae* vector according to the invention.

Another subject matter of the present invention relates to a composition, preferably a pharmaceutical composition, containing the *Poxviridae* vector of the invention and/or the cell of the invention. The pharmaceutical composition can be preferably a vaccine, an immune stimulant, or an immune therapeutic.

The characteristics, advantages, features, and further developments of the *Poxviridae* vector according to the invention apply to the cell of the invention and the composition of the invention in a corresponding manner.

Another subject-matter of the invention relates to a method for the treatment of a living being, preferably a mammalian, further preferably a human being, in need of a vaccination with an antigen, comprising the administration of the recombinant vector or the pharmaceutical composition according to the invention.

The characteristics, advantages, features, and further developments of the *Poxviridae* vector according to the invention apply to the method of the invention in a corresponding manner.

It is to be understood that the previously mentioned features and those to be explained in the following cannot only be used in the combination indicated in each case, but also in other combinations or in isolated position without departing from the scope of the invention.

The invention is now further explained by means of examples which result in additional features, advantages and characteristics of the invention. The examples are purely illustrative and do not restrict the scope of the invention. Reference is made to the enclosed figures.

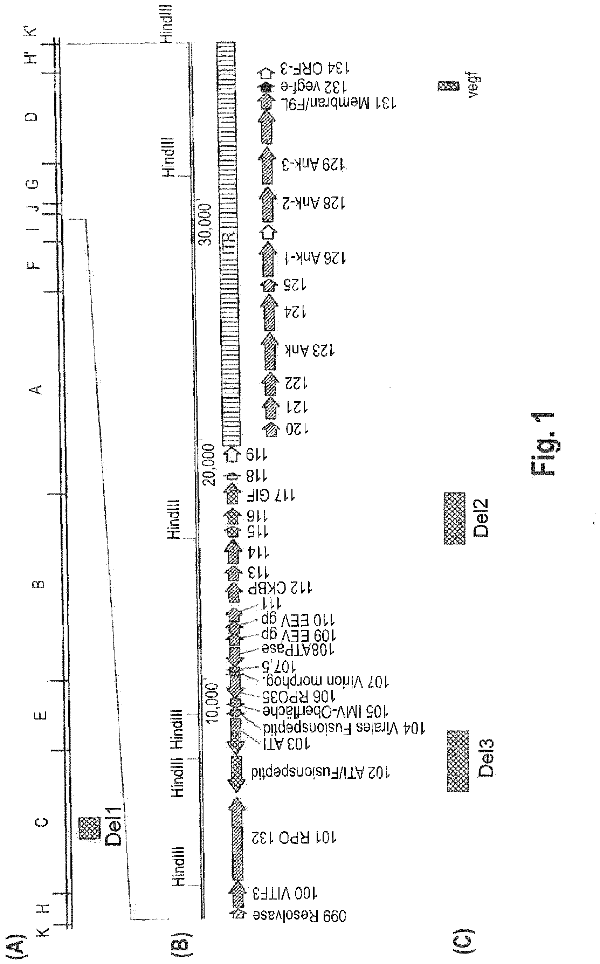

- Fig. 1: shows the map of the Hind *III* restriction fragments of the ORFV D1701-V DNA genome. The hatched boxes represent the insertion positions Del1, Del2, Del3 and vegf. ITRL stands for the inverted terminal repeats of the ends of the genome.

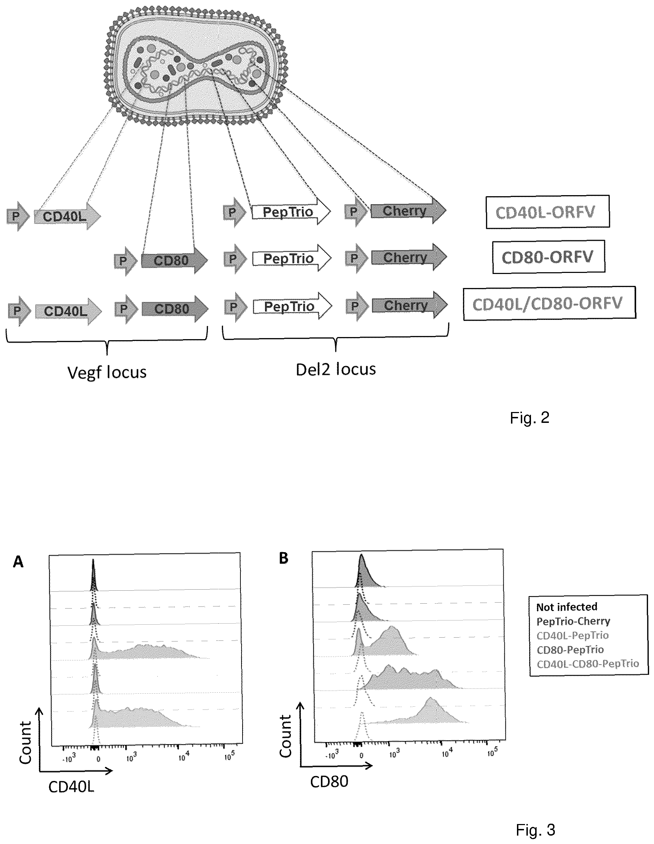

- Fig. 2: shows a schematic overview of the generated recombinants. CD40L and/or CD80 were integrated into the vegf locus. The artificial antigen "PepTrio" and the fluorescence marker protein mCherry are located in the Del2 locus.
- Fig. 3: shows the result of the analysis of ORFV infected DCs for expression of human transgenes. For the detection of transgene expression DCs were infected with PepTrio-ORFV, CD40L-PepTrio-ORFV, CD80-PepTrio-ORFV and CD40L-CD80-PepTrio-ORFV (MOI 1.0). After 24 h the expression of CD40L and CD80 were determined by flow cytometry after staining with specific antibodies. A) Expression of CD40L on the surface of infected DCs. B) Expression of CD80 on the surface of infected DCs.

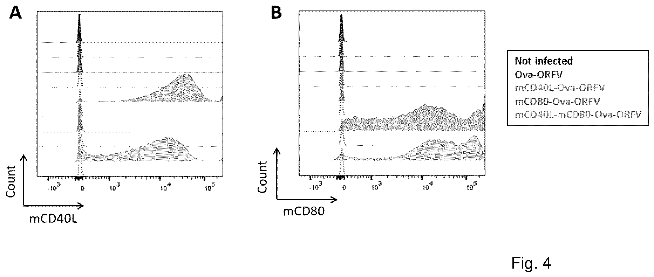

- Fig. 4: shows the result of the analysis of ORFV infected Vero cells for expression of murine transgenes. For the detection of transgene expression Vero cells were infected with Ova-ORFV, mCD40L-Ova-ORFV, mCD80-Ova-ORFV and mCD40L-mCD80-Ova-ORFV. After 24 h the expression of mCD40L and mCD80 were determined by flow cytometry after staining with specific antibodies. A) Expression of mCD40L on the surface of infected Vero cells. B) Expression of mCD80 on the surface of infected Vero cells.

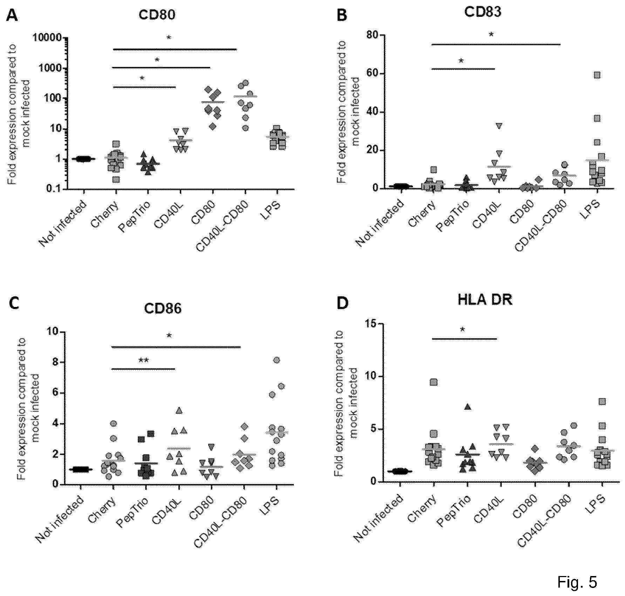

- Fig. 5: shows the activation of DCs from 8 different donors after infection with ORFV recombinants. DCs were infected with V-D12-Cherry, PepTrio-ORFV, CD40L-PepTrio-ORFV, CD80-PepTrio-ORFV and CD40L-CD80-PepTrio-ORFV and stained after 24 h with specific antibodies for the activation markers. A)-D) Relative expression strength of the activation markers CD80 (A), CD83 (B), CD86 (C) and HLA DR (D) after infection with the ORFV recombinants in relation to the expression strength on non-infected DCs. Shown is the change in expression level for each of the 8 independently performed experiments and the calculated mean values. The statistical analysis was performed by a paired t-test with a confidence interval of 95 %: ns = p > 0.5, * = p ≤ 0.05, ** = p ≤ 0.01.

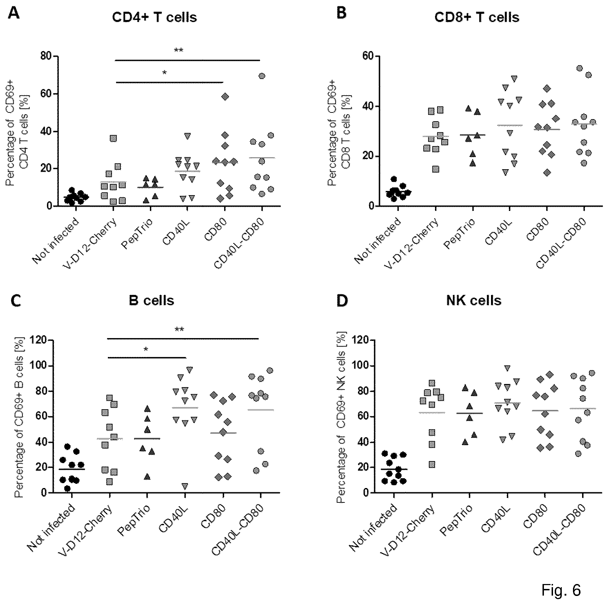

- Fig. 6:: shows the activation of human PBMCs from 10 different donors after infection with ORFV recombinants. PBMCs were isolated from the blood of 10 healthy donors and infected for 24 h with V-D12-Cherry, PepTrio-ORFV, CD40L-PepTrio-ORFV, CD80-PepTrio-ORFV and CD40L-CD80-PepTrio-ORFV. A)-D) Proportion of CD69+ cells after infection with ORFV recombinants in each experiment performed and the calculated mean values. Statistical analysis was performed by the non-parametric Wilcoxon sign rank test with a confidence interval of 95 %: ns = p > 0.5, * = p ≤ 0.05, * = p ≤ 0.01.

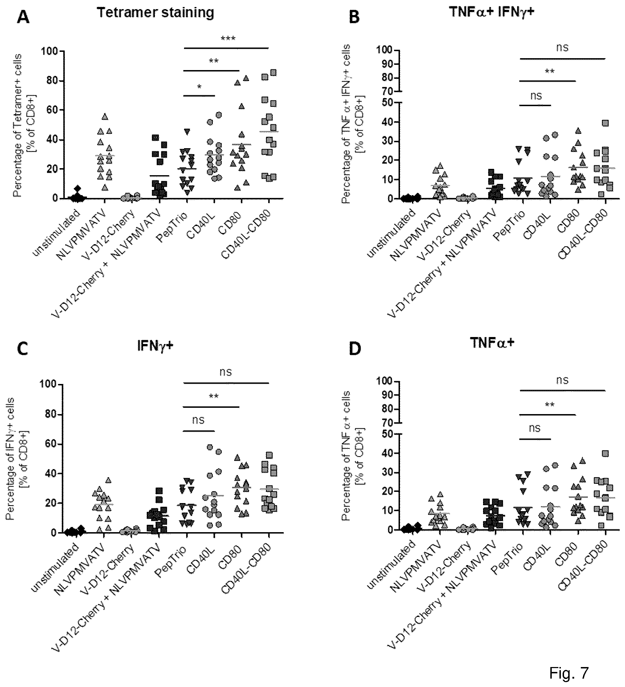

- Fig. 7:: shows the expansion and functionality of memory T cells from 7 different donors after infection with ORFV recombinants. PBMCs were stimulated with NLVPMVATV or infected with PepTrio-ORFV, CD40L-PepTrio-ORFV, CD80-PepTrio-ORFV and CD40L-CD80-PepTrio-ORFV (MOI 5.0). A) After 12 days the expansion of the memory T cells was determined by the proportion of Tetramer+ CD8+ T cells. B)-D) Functionality analysis was performed after re-stimulation with NLVPMVATV by intracellular staining of TNFα and IFNγ in CD8+ T cells. The percentage of TNFα+IFNγ+ CD8+ T cells (B), IFNγ+ CD8+ T cells (C) and TNFα+ CD8+ T cells (D) was determined. Shown are the duplicates of 7 independent experiments and the calculated mean values. The statistical analysis was performed by a paired t-test with a confidence interval of 95 %: ns = p > 0.5, * = p ≤ 0.05, ** = p ≤ 0.01, *** = p ≤ 0.001.

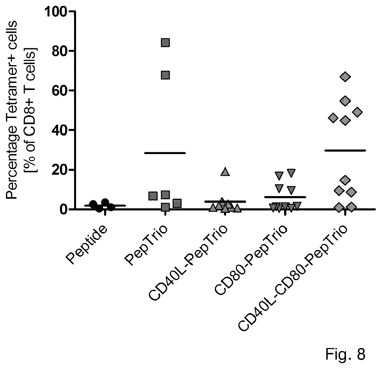

- Fig. 8: shows the priming of naïve T cells from four different donors using ORFV infected DCs. The figure shows the proportion of tetramer+ CD8+ T cells in the positive replicates.

### Examples

### 1. The ORFV genome

The ORFV genome consists of a linear double-stranded DNA and has a length of about 138 kB, a GC content of about 64 % and comprises 130-132 genes. The construction of the ORFV genome is similar to that of other pox viruses. It consists of a central region with essential genes which comprise a high degree of conservation within the pox viridae. In the ORFV genome there are 88 genes which are conserved in all Chordopoxvirinae. In the terminal regions viral genes are localized which are non-essential for the growth *in vitro,* however which are relevant for the pathogenicity and the tropism of the virus.

In comparison to other Orf viruses the D1701 virus which is adapted to the replication in cell culture shows a significant enlargement of the inverted terminal repeats (ITR). These alterations do not only cause a loss but also a duplication of several genes, including the vegf-e gene. The adaptation of D1701-B amplified in bovine BK-KL3A cells to the growth in Vero cells resulted in three additional insertion loci Del1, Del2 and Del3 in the virus genome which is now referred to as D1701-V. They are illustrated in the Figure 1.

### 2. Material and methods

### Primary human cells

For the isolation of PBMCs (Peripheral Blood Mononuclear Cells) blood samples from healthy donors from the blood donation center in Tübingen, Germany, were used.

The use of PBMCs from donor blood was approved by the Tübingen Ethics Commission: "Influence of Orf virus vectors on cells of the immune system". Number of the ethics application: 156/2012BO1.

### Isolation of PBMCs from blood

To isolate PBMCs from blood, the blood sample was diluted to a total volume of 100 ml with PBS. 25 ml of diluted blood was coated over 15 ml Ficoll in a 50 ml tube. The tubes were centrifuged for 20 min at 700 x g and RT. The leucocyte layer on the Ficoll was transferred to a 50 ml tube and made up to 50 ml with PBS. After centrifugation at 400 x g for 10 min, the cell pellet was resuspended in 50 ml PBS and centrifuged at 300 x g for 10 min. The cell pellets were then combined in PBS and the cell count was determined.

### Isolation of monocytes from PBMCs

The purification of monocytes from PBMCs was performed by CD14+ MACS selection. PBMCs were centrifuged for 10 min at 300 x g. The pellet was resuspended in 4 ml PBE and 100 µl α-CD14-MicroBeads (Miltenyi Biotech) were added. After incubation for 15 min at 4°C, the cells were loaded onto a LS column previously equilibrated with PBE (1 x PBS, 0.5 % (v/v) FCS, 5 mM EDTA). The column was washed three times with 3 ml PBE and the CD14+ monocytes were then eluted from the column with 5 ml PBE using a stamp. After elution, the cell count was determined.

### Cultivation of PBMCs and monocytes

Isolated PBMCs and purified monocytes were cultivated in IMDM (Gibco) with 10% (v/v) heat inactivated FCS and 0.2% (v/v) P/S at 37°C and 5% CO₂. Unless otherwise indicated, 5 x 10⁵ PBMCs and 1 x 10⁵ monocytes were seeded in a 96-well plate with U-bottom.

### Differentiation of monocytes into DCs

For the differentiation into DCs the purified monocytes were stimulated for 5 days with 86 ng/ml GM-CSF and 10 ng/ml IL-4. Cultivation was carried out in IMDM mixed with 10% (v/v) FCS and 0.2% (v/v) P/S at 37°C and 5% CO₂.

### Priming of naïve T cells with ORFV-infected DCs

ORFV recombinants expressing the HLA-A*02-restricted epitope pp65495-503 NLVPMVATV of human cytomegalovirus were used for priming of naïve T cells. PBMCs were isolated from the blood of donors with HLA-A*02 and the live cell count was determined. The total cell count should be approximately 1 x 10⁹. To find out whether NLVPMVATV-specific T-memory cells are present in the respective donor, a parallel ex vivo tetramer staining was performed. Additionally, 5 x 10⁶ PBMCs of the donor were stimulated with 1 µM NLVPMVATV peptide to exclude the expansion of possible NLVPMVATV-specific T-memory cells. For this purpose a tetramer staining was performed again after 5 days.

Half of the isolated PBMCs were stored in freezing medium (90 % FCS + 10 % DMSO) in a concentration of 1 x 10⁸/ml at -80°C. Monocytes were isolated from the remaining PBMCs and differentiated to DCs in a T175 cell culture flask with a concentration of 1-2 x 10⁶ cells/ml. The negative fraction of CD14+ MACS for isolation of the monocytes was stored in freezing medium at a concentration of 1 x 10⁸/ml at -80°C.

After 7 days the DCs were harvested and 2 x 10⁵ DCs/well/100 µl IMDM were seeded in a 96-well plate with U-bottom. Since peptide-loaded DCs served as positive control, a part of the DCs were stimulated with several cytokines for 24 h (10 ng/ml IL-4, 800 U/ml GM-CSF, 10 ng/ml LPS, 100 U/ml IFN-γ).

For the isolation of CD8+ T cells, the frozen PBMCs were thawed in thawing medium (RPMI + 0,003 mg/ml DNAse) and centrifuged for 10 min at 300 x g and RT. The cell pellet was resuspended in 4 ml PBE and 500 µl α-CD8-MicroBeads (Miltenyi Biotech) were added. After incubation at 4°C for 15 min the cells were transferred to a previously equilibrated LD column. After washing three times with 3 ml PBE each, the CD8+ T cells were eluted from the column with 5 ml PBE using a stamp. After determination of the live cell count the CD8+ T cells were incubated in TCM (RPMI with 5% heat inactivated human serum and 1% P/S) with 5 ng/ml IL-7 at a concentration of 3 x 10⁶ cells/ml overnight at 37°C. The CD8+ T-cells were then centrifuged for 10 min at RT and 300 x g and resuspended in TCM at a concentration of 1 x 10⁶ cells/100 µl with 5 ng/ml IL-12.

The immature DCs seeded in the 96-well plate were infected with MOI 5.0 using various ORFV recombinants for 6 h, the matured DCs were loaded with 25 µg/ml NLVPMVATV for 2 h. Subsequently, 1 x 10⁶ CD8+ T cells in 100 µl were added to the DCs.

The medium was changed every 2-3 days. For this purpose, 100 µl of medium were removed from each well and replaced by 100 µl TCM with 40 U/ml IL-2.

7 days after co-cultivation of DCs and CD8+ T cells were re-stimulated with autologous peptide-loaded PBMCs. Therefore, PBMCs of the respective donor were thawed. After thawing, PBMCs were resuspended in 1 ml TCM and the live cell count was determined. The PBMCs were then loaded with 25 µg/ml NLVPMVATV and incubated for 2 h at 37°C and 5% CO₂. For stimulation 1 x 106 peptide-loaded PBMCs were added to each well of the priming assay after irradiation with 30 Gy. In total, CD8+ cells were re-stimulated 3 or 4 times at intervals of 7 days.

7 days after the last re-stimulation, the priming was analyzed using tetramer staining.

### Expansion of antigen-specific T-memory cells

Since ORFV recombinants expressing the HLA-A*02-restricted epitope pp65495-503 NLVPMVATV of HCMV were used for the expansion of antigen-specific T-memory cells, PBMCs were isolated from the blood of donors with HLA-A*02. In order to find out whether NLVPMVATV-specific T-memory cells were present in the respective donor, a tetramer staining was performed ex vivo. After determination of the live cell count, 5 x 10⁶ PBMCs were seeded in 2 ml TCM per well of a 24-well plate and incubated for 6 h at 37°C and 5% CO₂. The PBMCs were infected with different ORFV recombinants or stimulated with 1 - 10 µM NLVPMVATV as a control. Every 2-3 days 500 µl of used medium was replaced by 500 µl TCM with 20 U/ml IL-2. After 12 days of stimulation, the analysis of the expansion was performed by tetramer staining and the analysis of the functionality of the antigen-specific T cells by intracellular cytokine staining.

### Flow cytometry

Flow cytometry was used to determine the viability and infection rates of infected cells and the expression of surface and intracellular molecules. Staining of the cells with fluorescence-labelled antibodies was performed in 96-well plates with U-bottom. The centrifugation steps were performed at 4°C and 400 x g for 5 min. For analysis the BD FACS LSRFortessa (BD Biosciences) was used.

### Inhibition of the Fc-receptor

To prevent the non-specific binding of antibodies to the Fc receptor, the cells were treated with Fc-Block (BioLegend) prior to antibody staining. The Fc-Block was used according to the manufacturer's instructions.

### Staining with multimers

Staining with tetramers was used to identify and quantify antigen-specific T cells. A tetramer consists of a fluorescence-labelled streptavidin complex that binds up to four biotinylated MHC molecules with high affinity. The MHC molecules contained in the tetramers carry an epitope of the respective antigen and form a peptide-MHC complex. T cells, which are specific for both the MHC and the bound epitope, can be detected in the flow cytometer after staining with tetramers.

Cells in a 96-well plate with U-bottom were washed twice with 200 µl PBS and resuspended in 50 µl tetramer solution. The tetramer solution contains 50 µl tetramer buffer and 1 µl PE-conjugated HLA tetramer and was centrifuged for 5 min at 4°C and 21,000 x g before staining. Incubation was performed at RT for 30 min in the dark.

### Determination of cell viability

To determine the viability of cells, staining with Zombia aqua (BioLegend) was performed. Zombie Aqua is an amine-reactive fluorescent dye that cannot penetrate the cell membrane of living cells. In dead cells the dye enters the cytoplasm and binds to intracellular proteins. The significantly higher fluorescence signal allows the differentiation of living and dead cells.

Cells in a 96-well plate with U-bottom were washed twice with 200 µl PBS and stained with 50 µl Zombie Aqua (diluted 1:400 in PBS) for 30 min at 4°C in the dark.

### Extracellular antibody staining

To analyse the expression of surface molecules, cells were stained with fluorescence-labelled antibodies.

Cells in a 96-well plate with U-bottom were washed twice with 200 µl PFEA, stained with 50 µl antibody mix in PFEA and incubated for 30 min at 4°C in the dark.

### Intracellular cytokine staining

To detect intracellular proteins, cells were permeabilised and stained with fluorescence-labelled antibodies. For the detection of cytokines the cells were stimulated for 12-15 h as indicated and treated with 10 µg/ml Brefeldin A to prevent the secretion of the proteins.

Cells in a 96-well plate with a U-bottom were twice washed with 200 µl PFEA and treated with 50 µl Cytofix/Cytoperm for 30 min at 4°C in the dark. After washing twice with 200 µl Permwash the cells were stained in the dark with 50 µl antibody mix in Permwash for 30 min at 4°C.

### Fixation of cells for flow cytometry

For fixation the stained cells were washed twice with 200 µl PFEA and then resuspended in 50 µl PFEA + 1 % formaldehyde. The analysis of the cells was performed within one week.

### 3. Results

The inventors investigated whether the ORFV-induced immune response can be enhanced by the additional expression of co-stimulatory molecules. For this purpose, ORFV recombinants were generated which, in addition to the artificial antigen "PepTrio", express either CD40L and/or CD80 (Figure 2). The in vitro characterization of these recombinants was then used to determine whether ORFV-induced activation of the immune system is enhanced. In addition, recombinants were produced that express the model antigen Ovalbumin (Ova) in combination with the murine homologues of CD40L (mCD40L) and/or CD80 (mCD80). These recombinants will be used to study the induction of antigen-specific immune responses and the effect of co-expression of CD40L and CD80 in vivo.

### Analysis of transgene expression

To demonstrate the expression of the inserted human transgenes, DCs were infected with PepTrio-ORFV, CD40L-PepTrio-ORFV, CD80-PepTrio-ORFV and CD40L-CD80-PepTrio-ORFV (MOI 1.0). After 24 h the cells were harvested and stained with antibodies specific for the surface markers CD40L and CD80. The expression of CD40L and CD80 was then analyzed by flow cytometry.

After infection with CD40L-PepTrio-ORFV and CD40L-CD80-PepTrio-ORFV the expression of CD40L was detected on the surface of the infected DCs (Figure 3 A). Since CD40L is not naturally expressed on DCs, no expression of CD40L was detected on uninfected DCs and after infection with PepTrio-ORFV and CD80-PepTrio-ORFV (Figure 3 A).

Infection of DCs with CD80-PepTrio-ORFV and CD40L-CD80-PepTrio-ORFV resulted in increased expression of CD80. Infection with PepTrio-ORFV or CD40L-PepTrio-ORFV also increased the expression of CD80 compared to non-infected cells (Figure 3 B). ORFV infection leads to activation of the DCs, which is reflected in the upregulation of CD80.

For the detection of expression of the murine transgenes, Vero cells were infected with Ova-ORFV, CD40L-Ova-ORFV, CD80-Ova-ORFV and CD40L-CD80-Ova-ORFV. After 24 h the cells were harvested and stained with antibodies specific for mCD40L and mCD80. Subsequently, the proportion of mCD40L+ and mCD80+ cells was determined by flow cytometry. The mCD40L-Ova-ORFV and mCD40L-mCD80-Ova-ORFV infected Vero cells showed a strong expression of mCD40L on the cell surface. Ova-ORFV- and mCD80-Ova-ORFV-infected cells as well as non-infected cells showed no expression of mCD40L (Figure 4 A).

Infection with mCD80-Ova-ORFV and mCD40L-mCD80-Ova-ORFV resulted in mCD80 expression of the infected cells. No mCD80 expression was observed after infection with Ova-ORFV and CD40L-Ova-ORFV. As expected, no mCD80 expression was detected on non-infected cells either (Figure 4 B).

### Activation of human DCs by ORFV infection

In the following it was analyzed whether enhanced immune responses are induced by the additional expression of CD40L and CD80 in the new generated recombinants. For this purpose, the activation of APCs by infection with CD40L-PepTrio-ORFV, CD80-PepTrio-ORFV and CD40L-CD80-PepTrio-ORFV was compared to the activation of APCs by infection with PepTrio-ORFV and V-D12-Cherry.

DCs from 8 different donors were infected with V-D12-Cherry, PepTrio-ORFV, CD40L-PepTrio-ORFV, CD80-PepTrio-ORFV and CD40L-CD80-PepTrio-ORFV. Non-infected cells were used as negative control. As positive control, the DCs were stimulated with LPS. After 24 h the cells were harvested and stained with antibodies specific for the activation markers CD80, CD83, CD86 and HLA-DR.

In Figure 4 the expression of the activation markers is shown as the relative strength of expression compared to non-infected cells. Compared to the expression of activation markers after infection with V-D12-Cherry, infection with PepTrio-ORFV did not lead to significant changes in the expression level of all analyzed markers (Figure 5 A-D).

Infection of the DCs with CD40L-PepTrio-ORFV and CD40L-CD80-PepTrio-ORFV induced an increased expression of the analyzed activation markers CD80, CD83, CD86 and HLA DR (Figure 5 A-D). Consequently, the DCs were more strongly activated by infection with these recombinants compared to V-D12-Cherry and PepTrio-ORFV-infected cells. Infection with CD80-PepTrio-ORFV resulted in a very strong expression of CD80 on the surface of infected DCs (Figure 5 A), but the expression of further activation markers was not increased.

### Activation of human PBMCs by ORFV infection

Since infection with CD40L- and CD80-expressing ORFV recombinants led to an increased activation of APCs, in the next step it was analyzed whether the activation of PBMCs can also be increased by the additional expression of CD40L and/or CD80. For this purpose PBMCs from 10 different donors were isolated and infected for 24 h with the ORFV recombinants V-D12-Cherry, PepTrio-ORFV, CD40L-PepTrio-ORFV, CD80-PepTrio-ORFV and CD40L-CD80-PepTrio-ORFV. After infection, the cells were stained specifically for the surface markers CD4, CD8, CD14, CD19 and CD56 and the activation marker CD69.

In comparison to V-D12-Cherry-infected PBMCs, no significant change in CD69 expression on T cells, B cells and NK cells after infection with PepTrio-ORFV was observed (Figure 6 A-D). In contrast, the proportion of CD69+ CD4+ T-cells was slightly increased after infection with CD40L-PepTrio-ORFV. After infection with CD80-PepTrio-ORFV or CD40L-CD80-PepTrio-ORFV the proportion of CD69+ CD4+ T cells increased significantly (Figure 6 A). Infection with both CD40L-PepTrio-ORFV and CD40L-CD80-PepTrio-ORFV resulted in an increased proportion of CD69+ B cells. In contrast, infection with CD80-PepTrio-ORFV did not induce increased activation of B cells (Figure 6 C). The proportion of CD69+ CD8+ T cells and CD69+ NK cells was not significantly increased by any of the tested recombinants (Figure 6 B and D).

### Induction of antigen-specific immune responses in vitro

Since the additional expression of the two co-stimulatory molecules results in an increased activation and cytokine production of APCs, the following study investigated whether CD40L-PepTrio-ORFV, CD80-PepTrio-ORFV and CD40L-CD80-PepTrio-ORFV induce stronger antigen-specific immune responses. For this purpose, the expansion of memory T cells and their functionality 12 days after infection with the various ORFV recombinants was examined and compared with PepTrio-ORFV infection. In addition, the ability to activate naïve T cells against the NLVPMVATV contained in the PepTrio antigen was tested (priming).

In order to determine the proportion of antigen-specific T cells, a tetramer staining is performed. Fluorescence-labelled tetramers are used to stain those cells whose T-cell receptor specifically recognizes the specific antigen. Flow cytometry is used to determine the proportion of antigen-specific T-cells. An increase in the population of antigen-specific T cells indicates that the cells have been specifically activated, which has led to their proliferation. The functionality of the antigen-specific T cells is determined by the production of the effector cytokines IFNγ and TNFα. For this purpose, T cells that have already been stimulated for 12 days are re-stimulated with the specific antigen and subsequently the production of the two cytokines is analyzed by intracellular staining with specific antibodies followed by flow cytometry.

To analyze the expansion and functionality of NLVPMVATV-specific memory T cells, PBMCs were infected with the different ORFV recombinants. Unstimulated PBMCs served as negative control. Stimulation with peptide was used as positive control. Infection with V-D12-Cherry without antigen and infection with V-D12-Cherry with additional peptide stimulation provided further controls to investigate the influence of ORFV on T cell expansion and functionality. After 12 days, the expansion and functionality of the memory T cells were analyzed. To determine the basic expression of cytokines, the cells were stimulated with a negative peptide.

In total, the expansion and functionality of memory T cells was analyzed in 7 donors. The analysis was performed in duplicates. Figure 6 shows the summary of the experiments. The tetramer staining showed that infection with CD40L-PepTrio-ORFV, CD80-PepTrio-ORFV and CD40L-CD80-PepTrio-ORFV significantly increased the proportion of NLVPMVATV-specific memory T cells compared to PepTrio-ORFV-infected cells (Figure 6 A). The largest proportion of tetramer+ CD8+ T cells was achieved by infection with CD40L-CD80-PepTrio-ORFV (Figure 6 A). Infection with V-D12-Cherry did not induce expansion of memory T cells. The proportion of IFNγ+TNFα+ CD8+ T cells, IFNγ+ CD8+ T cells and TNFα+ CD8+ T cells was increased by infection with CD80-PepTrio-ORFV and CD40L-CD80-PepTrio-ORFV (Figure 7 B, C and D). Infection with CD40L-PepTrio-ORFV also resulted in increased levels of IFNγ+ CD8+ T cells (Figure 7 C).

For the activation of naïve T cells (priming), DCs were infected with PepTrio-ORFV, CD40L-PepTrio-ORFV, CD80-PepTrio-ORFV and CD40L-CD80-PepTrio-ORFV (MOI 5.0) and co-cultivated with autologous CD8+ T cells. Peptide-loaded DCs which were previously stimulated with several cytokines served as control group. The unstimulated control shows the proportion of Tetramer+ CD8+ T cells, i.e. the proportion of antigen-specific T cells without stimulation with peptide or infection with the various ORFV recombinants. Priming of naïve T cells using CD40L-PepTrio-ORFV, CD80-PepTrio-ORFV and CD40L-CD80-PepTrio-ORFV was performed with the cells of four different donors. For each condition, approximately 20 replicates were used. A replicate was considered positive if the proportion of Tetramer+ cells after stimulation was at least 0.2% and at least twice as high as in the unstimulated control. Table 1 shows the percentage of positive replicates. The most successful priming was achieved by infection of the DCs with CD80-PepTrio-ORFV or CD40L-CD80-PepTrio-ORFV, followed by infection with CD40L-PepTrio-ORFV.

Looking at the percentage of tetramer+ CD8+ T cells in the positive replicates, the largest population of tetramer+ CD8+ T cells was obtained upon priming with CD40L-CD80-PepTrio-ORFV infected DCs. In summary, priming of naïve T cells was most successful after infection of the DCs with CD40L-CD80-PepTrio-ORFV (Figure 8).

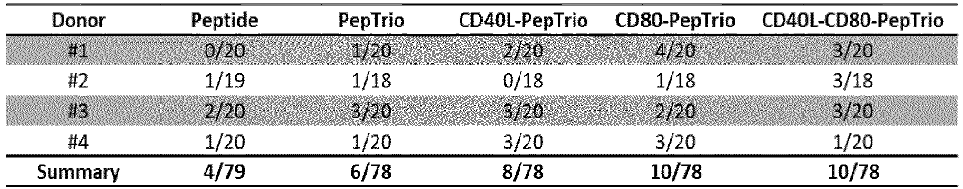

Table 1: Number of positive replicates after priming of naïve T cells. Shown is the number of tested replicates as well as the number of positive replicates from four donors.

Both the expansion of T-memory cells by infection of PBMCs and the priming of naïve T-cells by PepTrio-ORFV infected DCs showed that the ORFV-encoded antigen is expressed in APCs and presented on their cell surface. In addition, ORFV infection leads to an activation of the APCs, which are thus capable of inducing an antigen-specific CD8+ T cell response. The induction of the antigen-specific immune response was further enhanced in particular by the co-expression of CD40L and CD80.

### 4. Conclusion

In the frame of this work, viral vectors were generated that express the co-stimulatory, exemplified by molecules CD40L and CD80. The expression of CD40L caused a strong activation of APCs by binding to CD40, which was shown by the increased expression of different activation markers and the production of pro-inflammatory cytokines. The expression of CD80 enhanced the activation of T cells by binding to CD28. The combination of both molecules led to an increased induction of antigen-specific immune responses in vitro. The immunostimulatory properties of the ORFV vector D1701-V were successfully enhanced by the additional expression of the co-stimulatory molecules.

## Claims

1. A recombinant *Poxviridae* vector, which comprises the following:
- at least one first nucleotide sequence encoding and expressing a co-stimulatory molecule, and
- at least one promoter controlling the expression of the first nucleotide sequence.

2. The recombinant *Poxviridae* vector of claim 1, **characterized in that** the co-stimulatory molecule is CD40L.

3. The recombinant *Poxviridae* vector of claim 1, **characterized in that** the co-stimulatory molecule is CD80.

4. The recombinant *Poxviridae* vector of any of claims 1-3, which additionally comprises the following:
- at least a second nucleotide sequence encoding and expressing a co-stimulatory molecule, and
- at least one promoter controlling the expression of the second nucleotide sequence.

5. The recombinant *Poxviridae* vector of any of claims 1-4, **characterized in that** the co-stimulatory molecule is CD40L and/or CD80.

6. The recombinant *Poxviridae* vector of any of claims 1-5, which is a recombinant Parapoxvirus vector, preferably a recombinant Orf virus (ORFV) vector, further preferably the ORFV is of the strain D1701.

7. The recombinant *Poxviridae* vector of any of claims 1-6, **characterized in that** the first nucleotide sequence is located in the vegf locus.

8. The recombinant *Poxviridae* vector of any of claims 1-7, **characterized in that** the second nucleotide sequence is located in the vegf locus.

9. The recombinant *Poxviridae* vector of any of claims 1-8, which additionally comprises the following:
- at least one third nucleotide sequence encoding and expressing an antigen, and
- at least one promoter controlling the expression of the third nucleotide sequence.

10. The recombinant *Poxviridae* vector of claim 9, **characterized in that** the third nucleotide sequence is located in the Del2 locus.

11. The recombinant *Poxviridae* vector of claim 9 or 10, **characterized in that** the antigen is selected from the following group:
- viral antigen, preferably
rabies virus antigen, including glycoprotein (RabG);
influenza A antigen, including nucleoprotein (NP), hemagglutinin (HA), neuraminidase (NA);
SARS-CoV-2 antigen, including spike protein (S) or fragments thereof;
- tumor antigen, preferably viral tumor antigen, including HPV selective viral tumor antigen;
- tumor associated antigen, including viral tumor associated antigen, including HPV selective viral tumor-associated antigen;
- parasitic antigen, preferably plasmodium antigen;
- cytokine.

12. A cell containing the recombinant *Poxviridae* vector of any of claims 1-11.

13. A pharmaceutical composition containing the recombinant *Poxviridae* vector of any of claims 1-11.

14. The pharmaceutical composition of claim 13, which is a vaccine.

15. A method for the treatment of a living being in need of a vaccination with an antigen, comprising the administration of the recombinant vector of any of claims 9-11, or the pharmaceutical composition of claims 13 or 14.
